(19) Europäisches Patentamt
European Patent Office
Office européen des brevets

(11) Veröffentlichungsnummer: **0 379 871**
**A2**

(12) **EUROPÄISCHE PATENTANMELDUNG**

(21) Anmeldenummer: **90100245.1**

(22) Anmeldetag: **06.01.90**

(51) Int. Cl.5: **C07D 233/60, C07D 409/12, C07D 249/08, A01N 43/50**

(30) Priorität: **21.01.89 DE 3901723**

(43) Veröffentlichungstag der Anmeldung: **01.08.90 Patentblatt 90/31**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Schade, Gerold, Dr.**
**Hahnenweg 8**
**D-5000 Köln 80(DE)**
Erfinder: **Marhold, Albrecht, Dr.**
**Carl-Duisberg-Strasse 329**
**D-5090 Leverkusen 1(DE)**
Erfinder: **Brandes, Wilhelm, Dr.**
**Eichendorffstrasse 3**
**D-5653 Leichlingen(DE)**

(54) **Azolyl-Derivate.**

(57) Neue Azolyl-Derivate der Formel

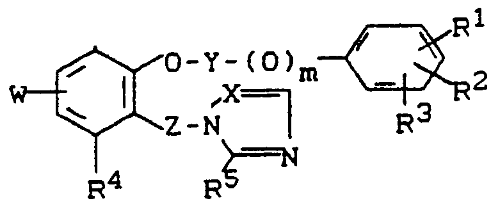

(I)

in welcher
$R^1$, $R^2$, $R^3$, $R^4$, $R^5$, W, X, Y, Z und m die in der Beschreibung angegebenen Bedeutungen haben, sowie deren Säureadditions-Salze und Metallsalz-Komplexe,
ein Verfahren zur Herstellung der neuen Stoffe und deren Verwendung als Mikrobizide.

EP 0 379 871 A2

## Azolyl-Derivate

Die vorliegende Erfindung betrifft neue Azolyl-Derivate, ein Verfahren zu deren Herstellung und deren Verwendung als Mikrobizide.

Es ist bereits bekannt, daß sich zahlreiche Benzylimidazol-Derivate zur Bekämpfung von Pilzen und Bakterien verwenden lassen (vgl. DE-OS 3 021 467, DE-OS 3 500 503 und EP-OS 0 162 359). So lassen sich z.B. 1-(1-[2-Thien-2-yl-methoxy)-phenyl]-vinyl)-imidazol und 1-(1-[2-(Thien-2-yl-methoxy)-phenyl]-2,2-dimethyl-vinyl)-imidazol als Fungizide gegen phytopathogene Pilze einsetzen. Bei niedrigen Aufwandmengen läßt die Wirksamkeit dieser Stoffe allerdings in manchen Fällen zu wünschen übrig.

Es wurden nun neue Azolyl-Derivate der Formel

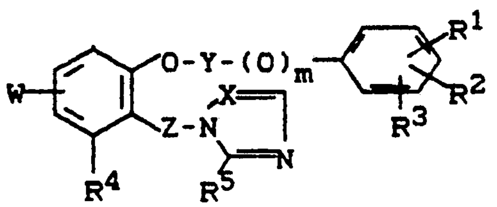

(I)

in welcher

$R^1$ für Halogenalkyl oder Halogenalkylthio steht,

$R^2$ für Wasserstoff, Halogen, Cyano, gegebenenfalls substituiertes Alkyl, gegebenenfalls substituiertes Alkenyl, Alkoxy, Alkylthio, Aryl oder Aryloxy steht,

$R^3$ für Wasserstoff, Halogen, Cyano, gegebenenfalls substituiertes Alkyl, gegebenenfalls substituiertes Alkenyl, Alkoxy, Alkylthio, Aryl oder Aryloxy steht,

$R^4$ für Wasserstoff steht,

$R^5$ für Wasserstoff oder Alkyl steht,

W für Wasserstoff oder Halogen steht,

X für Stickstoff oder eine CH-Gruppe steht,

Y für Alkylen steht,

m für die Zahlen 0 oder 1 steht und

Z für die Gruppierungen

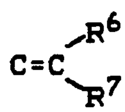 oder 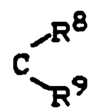

steht,

worin

$R^6$ für Wasserstoff, Alkyl oder gegebenenfalls substituiertes Phenyl steht oder

$R^6$ gemeinsam mit $R^4$ für die Gruppierung

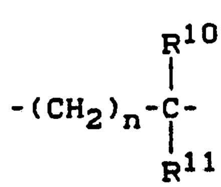

steht,

$R^7$ für Wasserstoff oder Alkyl steht,

$R^8$ für Wasserstoff oder Alkyl steht,

$R^9$ für Wasserstoff oder Alkyl steht,

$R^{10}$ für Wasserstoff oder Alkyl steht,

$R^{11}$ für Wasserstoff oder Alkyl steht und

n für die Zahlen 0, 1 oder 2 steht,
sowie deren Säureadditions-Salze und Metallsalz-Komplexe gefunden.

Weiterhin wurde gefunden, daß man Azolyl-Derivate der Formel (I) sowie deren Säureadditions-Salze und Metallsalz-Komplexe erhält, wenn man Phenol-Derivate der Formel

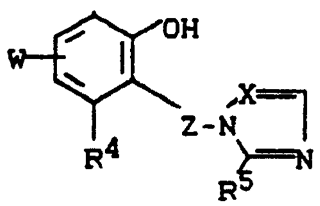

(II)

in welcher
$R^4$, $R^5$, W, X und Z die oben angegebene Bedeutung haben,
mit Basen gegebenenfalls in Gegenwart eines Verdünnungsmittels umsetzt und die dabei entstehenden Phenolate der Formel

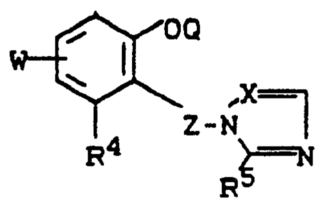

(II-a)

in welcher
$R^4$, $R^5$, W, X und Z die oben angegebene Bedeutung haben
und
Q für einen Baserest steht,
mit Halogenverbindungen der Formel

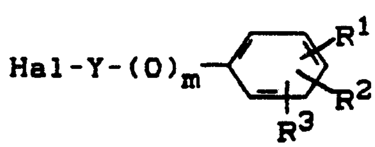

(III)

in welcher
$R^1$, $R^2$, $R^3$, Y und m die oben angegebene Bedeutung haben und
Hal für Chlor, Brom oder Iod steht,
in Gegenwart eines Verdünnungsmittels sowie gegebenenfalls in Gegenwart eines Säurebindemittels umsetzt und gegebenenfalls anschließend an die so erhaltenen Verbindungen der Formel (I) eine Säure oder ein Metallsalz addiert.

Außerdem wurde gefunden, daß die neuen Azolyl-Derivate der Formel (I) sowie deren Säureadditions-Salze und Metallsalz-Komplexe sehr gute mikrobizide Eigenschaften besitzen und im Pflanzenschutz eingesetzt werden können.

Überraschenderweise zeigen die erfindungsgemäßen Stoffe bei der Bekämpfung von phytopathogenen Pilzen eine deutlich bessere Wirksamkeit als 1-(1-[2-(Thien-2-yl-methoxy)-phenyl]-vinyl)-imidazol und 1-(1-[2-Thien-2-yl-methoxy)-phenyl]-2,2-dimethyl-vinyl)-imidazol, welches konstitutionell ähnliche vorbekannte Verbindungen gleicher Wirkungsrichtung sind.

Die erfindungsgemäßen Azolyl-Derivate sind durch die Formel (I) allgemein definiert. In dieser Formel stehen vorzugsweise
$R^1$ für Halogenalkyl mit 1 bis 4 Kohlenstoffatomen und 1 bis 5 gleichen oder verschiedenen Halogenatomen oder für Halogenalkylthio mit 1 bis 4 Kohlenstoffatomen und 1 bis 5 gleichen oder verschiedenen Halogenatomen,
$R^2$ für Wasserstoff, Fluor, Chlor, Brom, Cyano, gegebenenfalls durch Halogen substituiertes Alkyl mit 1 bis

6 Kohlenstoffatomen, gegebenenfalls durch Halogen substituiertes Alkenyl mit 2 bis 6 Kohlenstoffatomen, Alkoxy mit 1 bis 4 Kohlenstoffatomen, Alkylthio mit 1 bis 4 Kohlenstoffatomen, Aryl mit 6 bis 10 Kohlenstoffatomen oder Aryloxy mit 6 bis 10 Kohlenstoffatomen,
$R^3$ für Wasserstoff, Fluor, Chlor, Brom, Cyano, gegebenenfalls durch Halogen substituiertes Alkyl mit 1 bis 6 Kohlenstoffatomen, gegebenenfalls durch Halogen substituiertes Alkenyl mit 2 bis 6 Kohlenstoffatomen, Alkoxy mit 1 bis 4 Kohlenstoffatomen, Alkylthio mit 1 bis 4 Kohlenstoffatomen, Aryl mit 6 bis 10 Kohlenstoffatomen oder Aryloxy mit 6 bis 10 Kohlenstoffatomen,
$R^4$ für Wasserstoff,
$R^5$ für Wasserstoff oder Alkyl mit 1 bis 4 Kohlenstoffatomen,
W für Wasserstoff, Fluor, Chlor oder Brom,
X für Sickstoff oder eine CH-Gruppe,
Y für eine geradkettige oder verzweigte Alkylenkette mit 1 bis 6 Kohlenstoffatomen,
m für die Zahlen 0 oder 1 und
Z für die Gruppierungen

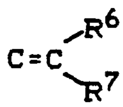 oder 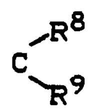 ,

worin
$R^6$ für Wasserstoff, Alkyl mit 1 bis 4 Kohlenstoffatomen oder für gegebenenfalls einfach bis dreifach, gleichartig oder verschieden durch Alkyl mit 1 bis 4 Kohlenstoffatomen, Halogen und/oder Halogenalkyl mit 1 oder 2 Kohlenstoffatomen und 1 bis 5 Halogenatomen substituiertes Phenyl steht oder
$R^6$ gemeinsam mit $R^4$ für die Gruppierung

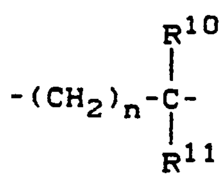 steht,

$R^7$ für Wasserstoff oder Alkyl mit 1 bis 4 Kohlenstoffatomen steht,
$R^8$ für Wasserstoff oder Alkyl mit 1 bis 4 Kohlenstoffatomen steht,
$R^9$ für Wasserstoff oder Alkyl mit 1 bis 4 Kohlenstoffatomen steht,
$R^{10}$ für Wasserstoff oder Alkyl mit 1 bis 4 Kohlenstoffatomen steht,
$R^{11}$ für Wasserstoff oder Alkyl mit 1 bis 4 Kohlenstoffatomen steht und
n für die Zahlen 0, 1 oder 2 steht.

Besonders bevorzugt sind diejenigen Verbindungen der Formel (I), in denen
$R^1$ für Halogenalkyl mit 1 oder 2 Kohlenstoffatomen und 1 bis 5 gleichen oder verschiedenen Halogenatomen, wie Fluor, Chlor und/oder Brom, steht oder für Halogenalkylthio mit 1 oder 2 Kohlenstoffatomen und 1 bis 5 gleichen oder verschiedenen Halogenatomen, wie Fluor, Chlor und/oder Brom, steht,
$R^2$ für Wasserstoff, Fluor, Chlor, Brom, Cyano, gegebenenfalls durch 1 bis 3 Fluor-, Chlor- und/oder Brom-Atome substituiertes Alkyl mit 1 bis 4 Kohlenstoffatomen, gegebenenfalls durch 1 bis 3 Fluor-, Chlor- und/oder Brom-Atome substituiertes Alkenyl mit 2 bis 4 Kohlenstoffatomen, Alkoxy mit 1 oder 2 Kohlenstoffatomen, Alkylthio mit 1 oder 2 Kohlenstoffatomen, Phenyl oder Phenoxy steht,
$R^3$ für Wasserstoff, Fluor, Chlor, Brom, Cyano, gegebenenfalls durch 1 bis 3 Fluor-, Chlor- und/oder Brom-Atome substituiertes Alkyl mit 1 bis 4 Kohlenstoffatomen, gegebenenfalls durch 1 bis 3 Fluor-, Chlor- und/oder Brom-Atome substituiertes Alkenyl mit 2 bis 4 Kohlenstoffatomen, Alkoxy mit 1 oder 2 Kohlenstoffatomen, Alkylthio mit 1 oder 2 Kohlenstoffatomen, Phenyl oder Phenoxy steht,
$R^4$ für Wasserstoff steht,
$R^5$ für Wasserstoff, Methyl oder Ethyl steht,
W für Wasserstoff oder Chlor steht,
X für Stickstoff oder eine CH-Gruppe steht,
Y für eine geradkettige oder verzweigte Alkylenkette mit 1 bis 4 Kohlenstoffatomen steht,
m für die Zahlen 0 oder 1 steht und
Z für die Gruppierungen

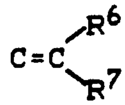 oder 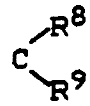

steht, worin
$R^6$ für Wasserstoff, Methyl, Ethyl, n-Propyl, Isopropyl, n-Butyl oder für gegebenenfalls einfach bis dreifach, gleichartig oder verschieden durch Methyl, Ethyl, Fluor, Chlor und/oder Trifluormethyl substituiertes Phenyl steht oder
$R^6$ gemeinsam mit $R^4$ für die Gruppierung

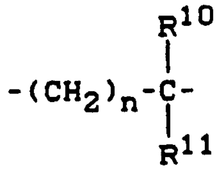 steht,

$R^7$ für Wasserstoff, Methyl, Ethyl, n-Propyl, Isopropyl oder n-Butyl steht,
$R^8$ für Wasserstoff, Methyl, Ethyl, n-Propyl, Isopropyl oder n-Butyl steht,
$R^9$ für Wasserstoff, Methyl, Ethyl, n-Propyl, Isopropyl oder n-Butyl steht,
$R^{10}$ für Wasserstoff oder Methyl steht,
$R^{11}$ für Wasserstoff oder Methyl steht und
n für die Zahlen 0 oder 1 steht.

Eine ganz besonders bevorzugte Gruppe von Verbindungen sind diejenigen Stoffe der Formel (I), in denen
m für die Zahl 0 steht,
$R^1$ für Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio oder Pentachlorethylthio steht,
$R^2$ für Wasserstoff, Fluor, Chlor, Brom, Cyano, Methyl, Ethyl, Trifluormethyl, Vinyl, Allyl, 2-Chlor-vinyl, Methoxy, Methylthio, Phenyl oder Phenoxy steht,
$R^3$ für Wasserstoff, Fluor, Chlor, Brom, Cyano, Methyl, Ethyl, Trifluormethyl, Vinyl, Allyl, 2-Chlor-vinyl, Methoxy, Methylthio, Phenyl oder Phenoxy steht,
$R^4$ für Wasserstoff steht,
$R^5$ für Wasserstoff, Methyl oder Ethyl steht,
W für Wasserstoff oder Chlor steht,
X für Stickstoff oder eine CH-Gruppe steht,
Y für Methylen, Ethylen, Propylen oder eine Gruppe der Formel

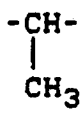

oder $-C(CH_3)_2-$ steht und
Z für die Gruppierungen

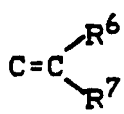 oder 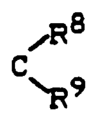

steht, worin

$R^6$ für Wasserstoff, Methyl, Ethyl, n-Propyl, Isopropyl, n-Butyl oder für gegebenenfalls einfach bis dreifach, gleichartig oder verschieden durch Methyl, Ethyl, Fluor, Chlor und/oder Trifluormethyl substituiertes Phenyl steht oder
$R^6$ gemeinsam mit $R^4$ für die Gruppierung

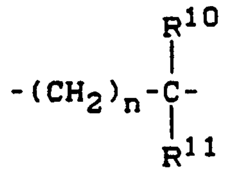

steht,

$R^7$ für Wasserstoff, Methyl, Ethyl, n-Propyl, Isopropyl oder n-Butyl steht,
$R^8$ für Wasserstoff, Methyl, Ethyl, n-Propyl, Isopropyl oder n-Butyl steht,
$R^9$ für Wasserstoff, Methyl, Ethyl, n-Propyl, Isopropyl oder n-Butyl steht,
$R^{10}$ für Wasserstoff oder Methyl steht,
$R^{11}$ für Wasserstoff oder Methyl steht und
n für die Zahlen 0 oder 1 steht.

Eine weitere Gruppe ganz besonders bevorzugter Verbindungen sind diejenigen Stoffe der Formel (I), in denen
m für die Zahl 1 steht,
$R^1$ für Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio oder Pentachlorethylthio steht,
$R^2$ für Wasserstoff, Fluor, Chlor, Brom, Cyano, Methyl, Ethyl, Trifluormethyl, Vinyl, Allyl, 2-Chlor-vinyl, Methoxy, Methylthio, Phenyl oder Phenoxy steht,
$R^3$ für Wasserstoff, Fluor, Chlor, Brom, Cyano, Methyl, Ethyl, Trifluormethyl, Vinyl, Allyl, 2-Chlor-vinyl, Methoxy, Methylthio, Phenyl oder Phenoxy steht,
$R^4$ für Wasserstoff steht,
$R^5$ für Wasserstoff, Methyl oder Ethyl steht,
W für Wasserstoff oder Chlor steht,
X für Stickstoff oder eine CH-Gruppe steht,
Y für Methylen, Ethylen, Propylen oder eine Gruppe der Formel

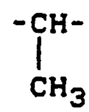

oder $-C(CH_3)_2-$ steht und
Z für die Gruppierungen

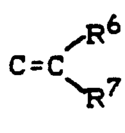 oder 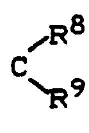

steht, worin
$R^6$ für Wasserstoff, Methyl, Ethyl, n-Propyl, Isopropyl, n-Butyl oder für gegebenenfalls einfach bis dreifach, gleichartig oder verschieden durch Methyl, Ethyl, Fluor, Chlor und/oder Trifluormethyl substituiertes Phenyl steht oder
$R^6$ gemeinsam mit $R^4$ für die Gruppierung

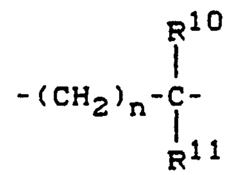 steht,

$R^7$ für Wasserstoff, Methyl, Ethyl, n-Propyl, Isopropyl oder n-Butyl steht,
$R^8$ für Wasserstoff, Methyl, Ethyl, n-Propyl, Isopropyl oder n-Butyl steht,
$R^9$ für Wasserstoff, Methyl, Ethyl, n-Propyl, Isopropyl oder n-Butyl steht,
$R^{10}$ für Wasserstoff oder Methyl steht,
$R^{11}$ für Wasserstoff oder Methyl steht und
n für die Zahlen 0 oder 1 steht.

Bevorzugte erfindungsgemäße Verbindungen sind auch Additionsprodukte aus Säuren und denjenigen Azolyl-Derivaten der Formel (I), in denen $R^1$, $R^2$, $R^3$, $R^4$, $R^5$, W, X, Y, Z und m diejenigen Bedeutungen haben, die bereits vorzugsweise für diese Reste bzw. den Index m genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Azolyl-Derivaten der Formel (I), in denen $R^1$, $R^2$, $R^3$, $R^4$, $R^5$, W, X, Y, Z und m diejenigen Bedeutungen haben, die bereits vorzugsweise für diese Reste bzw. den Index m genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Die erfindungsgemäßen Stoffe können in Form von cis- und trans-Isomeren vorliegen, wenn Z für die Gruppierung

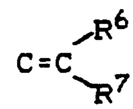

steht und $R^6$ und $R^7$ verschieden sind. Die Erfindung betrifft sowohl die reinen cis- und trans-Isomeren als auch deren Gemische.

Verwendet man 1-[1-(2-Hydroxy-phenyl)-vinyl]-imidazol als Ausgangsstoff, Natriumhydroxid als Base und 2-Chlor-4-trifluormethylthio-benzylchlorid als Reaktionskomponente, so kann der Verlauf des erfindungsgemäßen Verfahrens durch das folgende Formelschema veranschaulicht werden.

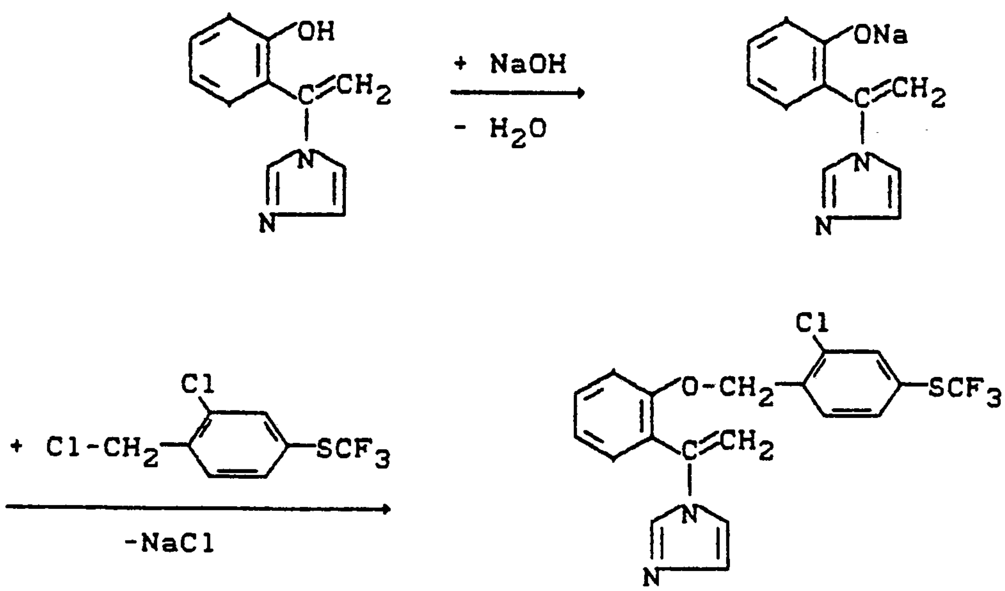


Die bei der Durchführung des erfindungsgemäßen Verfahrens als Ausgangsstoffe benötigten Phenol-Derivate sind durch die Formel (II) allgemein definiert. In dieser Formel haben $R^4$, $R^5$, W, X und Z vorzugsweise diejenigen Bedeutungen, die bereits im Zusammenhang mit der Beschreibung der erfindungsgemäßen Stoffe der Formel (I) vorzugsweise für diese Reste genannt wurden.

Die Phenol-Derivate der Formel (II) sind bekannt oder lassen sich nach prinzipiell bekannten Verfahren herstellen (vgl. J. Med. Chem. 27, 1142 (1984) und DE-OS 3 021 467).

Als Basen kommen bei der Durchführung des erfindungsgemäßen Verfahrens alle für derartige Umsetzungen üblichen starken Basen in Betracht. Vorzugsweise verwendbar sind Alkalimetallhydroxide, Alkalimetallamide, Alkalimetallalkoholate, Alkalimetallhydride, quarternäre Ammoniumhydroxide oder Phosphoniumhydroxide. Besonders bevorzugt sind Natriummethylat, Kalium-tert.-butylat, Natrium-amid, Natrium-hydrid und Tetramethylammonium-hydroxid. Demgemäß steht Q in der Formel (II-a) vorzugsweise für ein Alkalimetall-Kation, wie ein Natrium- oder Kaliumkation, oder für ein quarternäres Ammonium- oder Phosphonium-Kation.

Als Verdünnungsmittel kommen bei der Durchführung der ersten Stufe des erfindungsgemäßen Verfahrens alle für derartige Umsetzungen üblichen organischen Solventien in Frage. Vorzugsweise verwendbar sind Alkohole, wie Methanol, Ethanol, n-Propanal, Isopropanol, n-Butanol und tert.-Butanol.

Die Reaktionstemperaturen können bei der Durchführung der ersten Stufe des erfindungsgemäßen Verfahrens innerhalb eines größeren Bereiches variiert werden. Im allgemeinen arbeitet man bei Temperaturen zwischen 0° C und 100° C, vorzugsweise zwischen 10° C und 60° C.

Bei der Durchführung der ersten Stufe des erfindungsgemäßen Verfahrens arbeitet man im allgemeinen unter Normaldruck. Es ist jedoch auch möglich, unter erhöhtem oder vermindertem Druck zu arbeiten.

Bei der Durchführung der ersten Stufe des erfindungsgemäßen Verfahrens geht man im allgemeinen so vor, daß man auf 1 Mol an Phenol-Derivat 1 Mol an Base einsetzt. Es ist aber auch möglich, die eine oder andere Komponente in einem Überschuß einzusetzen. Die Aufarbeitung erfolgt nach üblichen Methoden. Im allgemeinen geht man so vor, daß man das Reaktionsgemisch durch Abziehen des Verdün nungsmittels einengt und das dabei verbleibende Phenolat entweder direkt oder nach vorheriger Reinigung für die weitere Synthese verwendet.

Die Halogenverbindungen, die bei der Durchführung der zweiten Stufe des erfindungsgemäßen Verfahrens als Reaktionskomponenten dienen, sind durch die Formel (III) allgemein definiert. In dieser Formel haben $R^1$, $R^2$, $R^3$, Y und m vorzugsweise diejenigen Bedeutungen, die bereits im Zusammenhang mit der Beschreibung der erfindungsgemäßen Stoffe der Formel (I) vorzugsweise für diese Reste bzw. den Index m genannt wurden. Hal steht für Chlor, Brom oder Iod.

Die Halogenverbindungen der Formel (III) sind bekannt (vgl. Synthesis 1983, 762 bis 763).

Die Halogenverbindung der Formel

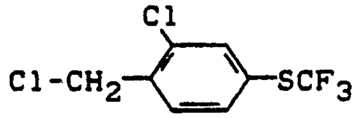

(III-1)

ist bisher noch nicht bekannt. Sie läßt sich herstellen, indem man 2-Chlor-4-trifluormethylthio-toluol der Formel

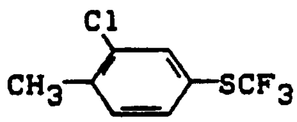

mit Chlor umsetzt.

Bei der Herstellung des 2-Chlor-4-trifluormethylthio-benzylchlorids der Formel (III-1) arbeitet man im allgemeinen unter vermindertem Druck, vorzugsweise unter einem Druck zwischen 20 und 120 mbar.

Die Temperaturen können bei der Herstellung des 2-Chlor-4-trifluormethylthio-benzylchlorids nach dem obigen Verfahren innerhalb eines bestimmten Bereiches variiert werden. Im allgemeinen arbeitet man bei Temperaturen zwischen 120° C und 200° C, vorzugsweise zwischen 140° C und 180° C.

Bei der Durchführung des Verfahrens zur Herstellung des 2-Chlor-4-trifluormethylthio-benzylchlorids der Formel (III-1) geht man im allgemeinen so vor, daß man 2-Chlor-4-trifluormethylthio-toluol mit einem Überschuß an Chlor versetzt und bei der jeweils gewünschten Temperatur unter dem jeweils gewünschten Druck reagieren läßt. Die Aufarbeitung erfolgt nach üblichen Methoden. Im allgemeinen geht man so vor, daß man das Reaktionsgemisch destilliert.

Als Verdünnungsmittel kommen bei der Durchführung der zweiten Stufe des erfindungsgemäßen Verfahrens alle für derartige Umsetzungen üblichen organischen Solventien in Betracht. Vorzugsweise verwendbar sind Alkohole, wie Methanol, Ethanol und Butanol, weiterhin Ether, wie Diethylether, Dioxan oder Tetrahydrofuran, ferner halogenierte aliphatische Kohlenwasserstoffe, wie Methylenchlorid, Chloroform oder Tetrachlorkohlenstoff, außerdem Nitrile, wie Acetonitril oder Propionitril, darüber hinaus Amide, wie Dimethylformamid, sowie stark polare Solventien, wie Dimethylsulfoxid oder Hexamethylphosphorsäuretriamid.

Als Säurebindemittel kommen bei der Durchführung der zweiten Stufe des erfindungsgemäßen Verfahrens alle üblichen Säureakzeptoren in Betracht. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kalium-tert.-butylat, außerdem tertiäre aliphatische oder aromatische Amine, wie Triethylamin, N,N-Dimethyl-cyclohexylamin, N,N-Dimethyl-benzylamin und Pyridin, und außerdem cyclische Amine, wie 1,5-Diaza-bicyclo[4.3.0]-non-5-en (DBN), 1,8-Diaza-bicyclo[5.4.0]undec-7-en (DBU) und 1,4-Diaza-bicyclo[2.2.2]octan (DABCO).

Die Reaktionstemperaturen können bei der Durchführung der zweiten Stufe des erfindungsgemäßen Verfahrens innerhalb eines größeren Bereiches variiert werden. Im allgemeinen arbeitet man bei Temperaturen zwischen 0° C und 120° C, vorzugsweise zwischen 10° C und 100° C.

Bei der Durchführung der zweiten Stufe des erfindungsgemäßen Verfahrens arbeitet man im allgemeinen unter Normaldruck. Es ist aber auch möglich, unter erhöhtem oder vermindertem Druck zu arbeiten.

Bei der Durchführung der zweiten Stufe des erfindungsgemäßen Verfahrens setzt man auf 1 Mol an Phenolat der Formel (II-a) im allgemeinen 1 bis 1,3 Mol an Halogenverbindung der Formel (III) ein. Die Aufarbeitung erfolgt nach üblichen Methoden. Im allgemeinen geht man so vor, daß man das Reaktionsgemisch durch Abziehen des Verdünnungsmittels einengt und den verbleibenden Rückstand durch Umkristallisation oder auf chromatographischem Wege reinigt.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten

organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide im Pflanzenschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Stoffe zeigen im Pflanzenschutz eine besonders gute Wirkung gegen Leptosphaeria nodorum sowie gegen Pyricularia oryzea. Außerdem besitzen sie starke bakterizide Eigenschaften und erweisen sich im Agarplattentest als sehr wirksam.

Die erfindungemäßen Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von0,0001 bis 0,02 %, am Wirkungsort erforderlich.

Die Herstellung und die Verwendung der erfindungsgemäßen Wirkstoffe wird durch die folgenden Beispiele veranschaulicht.

Herstellungsbeispiele

Beispiel 1

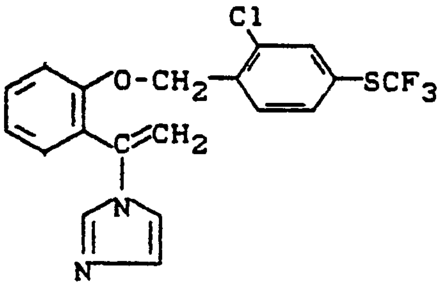

(I-1)

In ein Gemisch aus 7,44 g (0,04 Mol) 1-[1-(2-Hydroxyphenyl)-vinyl]-imidazol und 100 ml Methanol werden bei Raumtemperatur unter Rühren 1,6 g (0,04 Mol) Natriumhydroxid gegeben. Nach beendeter Zugabe wird noch 30 Minuten bei 50 °C gerührt und dann durch Abziehen des Lösungsmittels unter vermindertem Druck eingeengt. Der verbleibende feste Rückstand wird in 100 ml Dimethylformamid aufgenommen. In die entstehende Lösung werden 11,5 g (0,044 Mol) 2-Chlor-4-trifluormethylthiobenzylchlorid getropft. Nach zweistündigem Erhitzen auf 100 °C wird aufgearbeitet, indem durch Abziehen des Verdünnungsmittels eingeengt wird. Der anfallende Rückstand wird über Kieselgel mit Toluol/Essigester = 1:1 chromatographiert. Man erhält auf diese Weise 14,6 g (80,1 % der Theorie) an 1-[1-(2-{2-Chlor-4-trifluormethylthiobenzyloxy}-phenyl)-vinyl]-imidazol in Form einer Festsubstanz vom Schmelzpunkt 67 °C.

Herstellung der Ausgangssubstanz der Formel

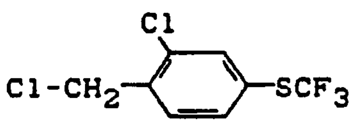

(III-1)

260 g (1,15 Mol) 3-Chlor-4-(methyl)-trifluorthiomethylbenzol werden in einem Rundkolben, der mit Vigreuxkolonne, Kolonnenkopf und gekühlter Vorlage versehen ist, zum Rückfluß erhitzt (100 % Rückfluß). Hierbei wird ein langsamer Chlorstrom über den Kolonnenkopf in die Dampfphase eingeleitet. Bei 60 %igem Umsatz wird das Reaktionsgemisch über eine 1 m Drehbandkolonne destilliert. Man erhält 151 g an 3-Chlor-4-(chlormethyl)-trifluorthio-methyl-benzol in 88 %iger Ausbeute (bezogen auf Umsatz).
Kp = 100 - 102 °C / 12 mbar
$n_D^{20}$ = 1,5240

Beispiel 2

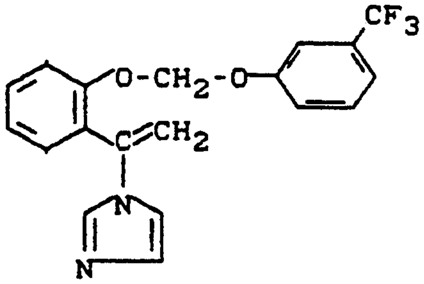

(I-2)

In ein Gemisch aus 7,4 g (0,04 Mol) 1-[1-(2-Hydroxyphenyl)-vinyl]-imidazol und 100 ml Methanol werden bei Raumtemperatur unter Rühren 1,6 g (0,04 Mol) Natriumhydroxid gegeben. Nach beendeter Zugabe wird noch 30 Minuten bei 50 °C gerührt und dann durch Abziehen des Lösungsmittels unter vermindertem Druck eingeengt. Der verbleibende feste Rückstand wird in 100 ml Dimethylformamid

aufgenommen. In die entstehende Lösung werden 9,2 g (0,044 Mol) 1-Chlormethyl-3-trifluormethylbenzol getropft. Nach einstündigem Erhitzen des Reaktionsgemisches auf 100 °C wird aufgearbeitet, indem man das Verdünnungsmittel unter vermindertem Druck abzieht. Der anfallende Rückstand wird über Kieselgel mit Toluol chromatographiert. Man erhält auf diese Weise 8,8 g eines öligen Produktes, das gemäß HPLC zu 94,6 % aus 1-[1-(2-{3-Trifluormethyl-phenoxy-methoxy}-phenyl)-vinyl]-imidazol besteht. Die Ausbeute errechnet sich danach zu 53,8 % der Theorie.
$^1$H-NMR-Spektrum (DMSO, TMS); 250 MHz
$\delta$ : 4,9 und 5,5 ppm (s)

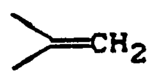

$\delta$ : 5,8 ppm (s) $-O-CH_2-O-$

Beispiel 3

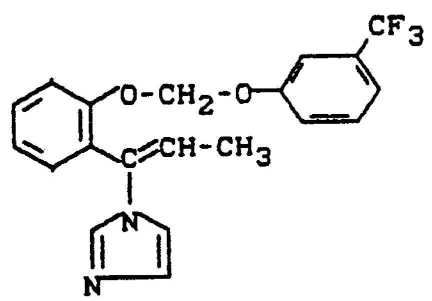

(I-3)

Nach den in den Beispielen 1 und 2 angegebenen Methoden wird auch die Verbindung der Formel (I-3) hergestellt. Man erhält die Verbindung in Form eines Öles. Die angegebene Struktur wird durch das Kernresonanzspektrum bestätigt. Es handelt sich um ein cis/trans-Gemisch.
$^1$H-NMR-Spektrum (DMSO, TMS); 250 MHz
$\delta$ : 1,35 (d) und 1,6 ppm (d) $-CH_3$
$\delta$ : 5,79 und 5,85 ppm (s) $-O-CH_2-O-$

In den folgenden Verwendungsbeispielen wurden die nachstehend angegebenen Verbindungen als Vergleichssubstanzen eingesetzt.

(A) = 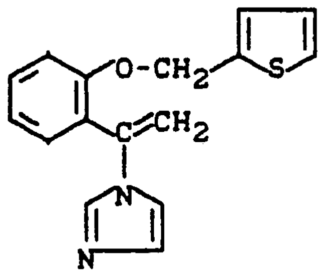

(B) = 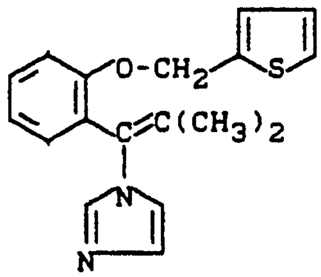

Beispiel A

Botrytis-Test (Bohne)/protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

In diesem Test zeigen die erfindungsgemäßen Stoffe (I-1), (I-2) und (I-3) eine wesentlich bessere Wirksamkeit als die Vergleichssubstanzen A und B.

Beispiel B

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit eine Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen

Luftfeuchtigkeit von ca. 80 % aufgestellt.

7 Tage nach der Inokulation erfolgt die Auswertung.

In diesem Test zeigen die erfindungsgemäßen Stoffe (I-1), (I-2) und (I-3) eine wesentlich bessere Wirksamkeit als die Vergleichssubstanzen (A) und (B).

Beispiel C

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome.

In diesem Test zeigen die erfindungsgemäßen Stoffe (I-2) und (I-3) eine wesentlich bessere Wirksamkeit als die Vergleichssubstanz (B).

## Ansprüche

1. Azolyl-Derivate der Formel (I)

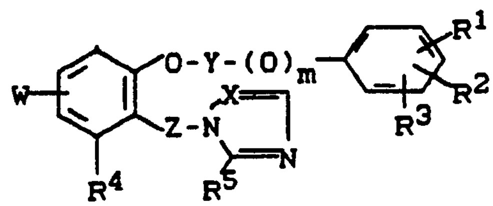

(I)

in welcher

$R^1$ für Halogenalkyl oder Halogenalkylthio steht,

$R^2$ für Wasserstoff, Halogen, Cyano, gegebenenfalls substituiertes Alkyl, gegebenenfalls substituiertes Alkenyl, Alkoxy, Alkylthio, Aryl oder Aryloxy steht,

$R^3$ für Wasserstoff, Halogen, Cyano, gegebenenfalls substituiertes Alkyl, gegebenenfalls substituiertes Alkenyl, Alkoxy, Alkylthio, Aryl oder Aryloxy steht,

$R^4$ für Wasserstoff steht,

$R^5$ für Wasserstoff oder Alkyl steht,

W für Wasserstoff oder Halogen steht,

X für Stickstoff oder eine CH-Gruppe steht,

Y für Alkylen steht,

m für die Zahlen 0 oder 1 steht und

Z für die Gruppierungen

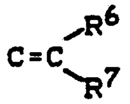 oder 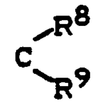 steht,

worin

$R^6$ für Wasserstoff, Alkyl oder gegebenenfalls substituiertes Phenyl steht oder

$R^6$ gemeinsam mit $R^4$ für die Gruppierung

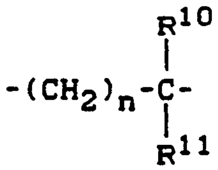 steht,

$R^7$ für Wasserstoff oder Alkyl steht,
$R^8$ für Wasserstoff oder Alkyl steht,
$R^9$ für Wasserstoff oder Alkyl steht,
$R^{10}$ für Wasserstoff oder Alkyl steht,
$R^{11}$ für Wasserstoff oder Alkyl steht und
n für die Zahlen 0, 1 oder 2 steht,
sowie deren Säureadditions-Salze und Metallsalz-Komplexe.

2. Azolyl-Derivate der Formel (I) gemäß Anspruch 1, in denen
$R^1$ für Halogenalkyl mit 1 oder 2 Kohlenstoffatomen und 1 bis 5 gleichen oder verschiedenen Halogenatomen steht oder für Halogenalkylthio mit 1 oder 2 Kohlenstoffatomen und 1 bis 5 gleichen oder verschiedenen Halogenatomen steht
$R^2$ für Wasserstoff, Fluor, Chlor, Brom, Cyano, gegebenenfalls durch 1 bis 3 Fluor-, Chlor-und/oder Brom-Atome substituiertes Alkyl mit 1 bis 4 Kohlenstoffatomen, gegebenenfalls durch 1 bis 3 Fluor-, Chlor- und/oder Brom-Atome substituiertes Alkenyl mit 2 bis 4 Kohlenstoffatomen, Alkoxy mit 1 oder 2 Kohlenstoffatomen, Alkylthio mit 1 oder 2 Kohlenstoffatomen, Phenyl oder Phenoxy steht,
$R^3$ für Wasserstoff, Fluor, Chlor, Brom, Cyano, gegebenenfalls durch 1 bis 3 Fluor-, Chlor-und/oder Brom-Atome substituiertes Alkyl mit 1 bis 4 Kohlenstoffatomen, gegebenenfalls durch 1 bis 3 Fluor-, Chlor- und/oder Brom-Atome substituiertes Alkenyl mit 2 bis 4 Kohlenstoffatomen, Alkoxy mit 1 oder 2 Kohlenstoffatomen, Alkylthio mit 1 oder 2 Kohlenstoffatomen, Phenyl oder Phenoxy steht,
$R^4$ für Wasserstoff steht,
$R^5$ für Wasserstoff, Methyl oder Ethyl steht,
W für Wasserstoff oder Chlor steht,
X für Stickstoff oder eine CH-Gruppe steht,
Y für eine geradkettige oder verzweigte Alkylenkette mit 1 bis 4 Kohlenstoffatomen steht,
m für die Zahlen 0 oder 1 steht und
Z für die Gruppierungen

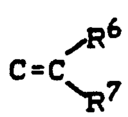 oder 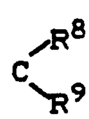

steht,
worin
$R^6$ für Wasserstoff, Methyl, Ethyl, n-Propyl, Isopropyl, n-Butyl oder für gegebenenfalls einfach bis dreifach, gleichartig oder verschieden durch Methyl, Ethyl, Fluor, Chlor und/oder Trifluormethyl substituiertes Phenyl steht oder
$R^6$ gemeinsam mit $R^4$ für die Gruppierung

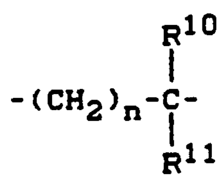 steht,

$R^7$ für Wasserstoff, Methyl, Ethyl, n-Propyl, Isopropyl oder n-Butyl steht,
$R^8$ für Wasserstoff, Methyl, Ethyl, n-Propyl, Isopropyl oder n-Butyl steht,
$R^9$ für Wasserstoff, Methyl, Ethyl, n-Propyl, Isopropyl oder n-Butyl steht,

$R^{10}$ für Wasserstoff oder Methyl steht,
$R^{11}$ für Wasserstoff oder Methyl steht und
n für die Zahlen 0 oder 1 steht.

3. Azolyl-Derivat gemäß Anspruch 1, gekennzeichnet durch die Formel

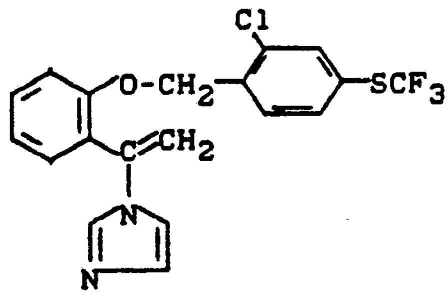

4. Azolyl-Derivat gemäß Anspruch 1, gekenzeichnet durch die Formel

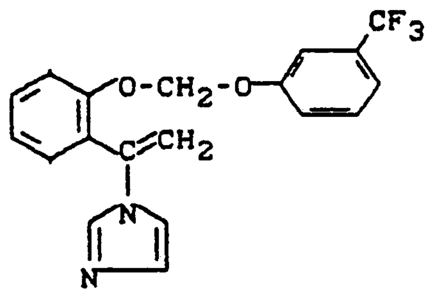

5. Azolyl-Derivat gemäß Anspruch 1, gekenzeichnet durch die Formel

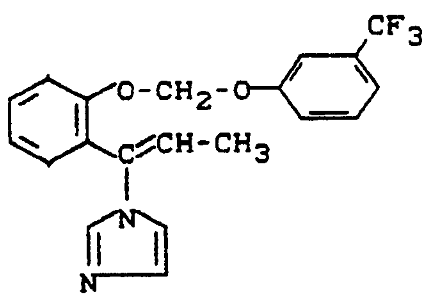

6. Verfahren zur Herstellung von Azolyl-Derivaten der Formel

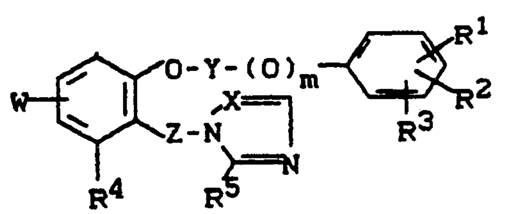

(I)

in welcher
$R^1$ für Halogenalkyl oder Halogenalkylthio steht,
$R^2$ für Wasserstoff, Halogen, Cyano, gegebenenfalls substituiertes Alkyl, gegebenenfalls substituiertes Alkenyl, Alkoxy, Alkylthio, Aryl oder Aryloxy steht,
$R^3$ für Wasserstoff, Halogen, Cyano, gegebenenfalls substituiertes Alkyl, gegebenenfalls substituiertes Alkenyl, Alkoxy, Alkylthio, Aryl oder Aryloxy steht,
$R^4$ für Wasserstoff steht,

$R^5$ für Wasserstoff oder Alkyl steht,
W für Wasserstoff oder Halogen steht,
X für Stickstoff oder eine CH-Gruppe steht,
Y für Alkylen steht,
m für die Zahlen 0 oder 1 steht und
Z für die Gruppierungen

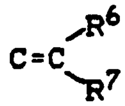 oder 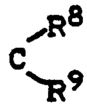 steht,

worin
$R^6$ für Wasserstoff, Alkyl oder gegebenenfalls substituiertes Phenyl steht oder
$R^6$ gemeinsam mit $R^4$ für die Gruppierung

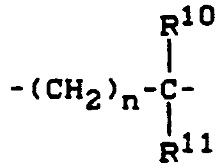 steht,

$R^7$ für Wasserstoff oder Alkyl steht,
$R^8$ für Wasserstoff oder Alkyl steht,
$R^9$ für Wasserstoff oder Alkyl steht,
$R^{10}$ für Wasserstoff oder Alkyl steht,
$R^{11}$ für Wasserstoff oder Alkyl steht und
n für die Zahlen 0, 1 oder 2 steht,
sowie von deren Säureadditions-Salzen und Metallsalz-Komplexen, dadurch gekennzeichnet, daß man Phenol-Derivate der Formel

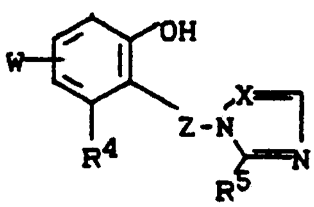 (II)

in welcher
$R^4$, $R^5$, W, X und Z die oben angegebene Bedeutung haben,
mit Basen gegebenenfalls in Gegenwart eines Verdünnungsmittels umsetzt und die dabei entstehenden Phenolate der Formel

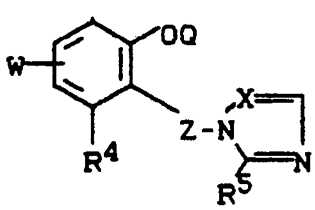 (II-a)

in welcher
$R^4$, $R^5$, W, X und Z die oben angegebene Bedeutung haben
und
Q für einen Baserest steht,

mit Halogenverbindungen der Formel

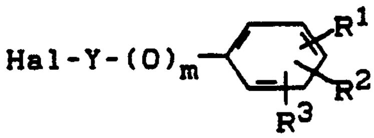

(III)

in welcher
$R^1$, $R^2$, $R^3$, Y und m die oben angegebene Bedeutung haben und
Hal für Chlor, Brom oder Iod steht,
in Gegenwart eines Verdünnungsmittels sowie gegebenenfalls in Gegenwart eines Säurebindemittels umsetzt und gegebenenfalls anschließend an die so erhaltenen Verbindungen der Formel (I) eine Säure oder ein Metallsalz addiert.

7. Mikrobizide Mittel, gekennzeichnet durch einen Gehalt an mindestens einem Azolyl-Derivat der Formel (I) gemäß Anspruch 1 bzw. an einem Säureadditions-Salz oder Metallsalz-Komplex eines Azolyl-Derivates der Formel (I).

8. Verwendung von Azolyl-Derivaten der Formel (I) gemäß Anspruch 1 bzw. von deren Säureadditions-Salzen und Metallsalz-Komplexen als Mikrobizide.

9. Verfahren zur Bekämpfung von unerwünschten Mikroorganismen, dadurch gekennzeichnet, daß man Azolyl-Derivate der Formel (I) gemäß Anspruch 1 bzw. deren Säureadditions-Salze oder Metallsalz-Komplexe auf die Mikroorganismen und/oder deren Lebensraum ausbringt.

10. Verfahren zur Herstellung von mikrobiziden Mitteln, dadurch gekennzeichnet, daß man Azolyl-Derivate der Formel (I) gemäß Anspruch 1 bzw. deren Säureadditions-Salze oder Metallsalz-Komplexe mit Streckmitteln und/oder oberflächenaktiven Stoffen vermischt.